# EUROPEAN PATENT SPECIFICATION

(11) **EP 2 645 878 B1**
(45) Date of publication and mention of the grant of the patent: **18.10.2017**
(21) Application number: 11794255.7
(22) Date of filing: 01.12.2011
(51) Int. Cl.: A23L 33/10, A23L 33/19, A23L 33/185, A61K 31/7016

(54) **PREVENTION OR TREATMENT OF OVERWEIGHT AND OBESITY IN TYPE 2 DIABETIC PATIENTS**
PRÄVENTION ODER BEHANDLUNG VON ÜBERGEWICHT UND FETTLEIBIGKEIT BEI PATIENTEN MIT TYP 2-DIABETES
PRÉVENTION OU TRAITEMENT DU SURPOIDS ET DE L'OBÉSITÉ CHEZ DES PATIENTS ATTEINTS D'UN DIABÈTE DE TYPE 2

(30) Priority: 01.12.2010 WO PCT/NL2010/050808
(43) Date of publication of application: 09.10.2013
(73) Proprietor: N.V. Nutricia, 2712 HM Zoetermeer (NL)
(72) Inventor: HAGEMAN, Robert Johan Joseph, NL-6705 CT Wageningen (NL); LANSINK, Mirian, NL-6704 PH Wageningen (NL)
(74) Representative: Nederlandsch Octrooibureau
(86) International application number: PCT/NL2011/050826
(87) International publication number: WO 2012/074401

(56) References cited:
- EP-A1- 1 424 074
- WO-A1-2006/087232
- WO-A1-2010/104374
- WO-A2-2006/009437
- WO-A2-2007/004883
- DE-A1-102006 017 611
- HAGEMAN ROBERT ET AL: "A specific blend of intact protein rich in aspartate has strong postprandial glucose attenuating properties in rats", JOURNAL OF NUTRITION, WISTAR INSTITUTE OF ANATOMY AND BIOLOGY, PHILADELPHIA, PA, US, vol. 138, no. 9, 1 September 2008 (2008-09-01), pages 1634-1640, XP002555472, ISSN: 0022-3166
- HABERER D ET AL: "Beneficial effects on glucose metabolism of chronic feeding of isomaltulose versus sucrose in rats", ANNALS OF NUTRITION AND METABOLISM: EUROPEAN JOURNAL OF NUTRITION, METABOLIC DISEASES AND DIETETICS, S. KARGER AG, SWITZERLAND, vol. 54, no. 1, 1 January 2009 (2009-01-01), pages 75-82, XP009125552, ISSN: 0250-6807
- KAWAI K ET AL: "Usefulness of palatinose as a coloric sweetener for diabetic patients", HORMONE AND METABOLIC RESEARCH, THIEME-STRATTON, STUTTGART, DE, vol. 21, no. 6, 24 February 1989 (1989-02-24), pages 338-340, XP002959842, ISSN: 0018-5043

## Description

### FIELD OF THE INVENTION

The invention rests in the field of diabetes, particularly type 2 diabetes patients. The invention particularly pertains to the dietary management of cholesterol levels and/or triglyceride levels in type 2 diabetes patients.

### BACKGROUND OF THE INVENTION

Obesity and type 2 diabetes represent a serious threat to the health of the population of almost every country in the world. The World Health Organization estimates that diabetes causes about 5% of all deaths globally each year, and this is almost certainly an underestimate. Additionally, diabetes deaths are likely to increase by more than 50% in the next 10 years without urgent action.

This escalation is due in part to increasing rates of childhood obesity. Until recently, type 2 diabetes was a disease that afflicted only adults. Nowadays, an increasing number of children are diagnosed with obesity-related type 2 diabetes. Also increasing is the incidence of 'metabolic syndrome' - a complex condition linked to obesity that is characterized by a cluster of closely related clinical features, including insulin resistance, dyslipidaemia and hypertension. Metabolic syndrome is associated with an increased risk of cardiovascular disease, which is ultimately responsible for a considerable proportion of diabetic mortality.

Many type-2 diabetes patients have some form of overweight and associated insulin resistance.

WO2006/009437 discloses the use of a composition for regulating blood glucose levels in diabetes patients. The composition is specifically used for flattening glucose response after eating. On page 37 lines 5-8 it is disclosed that in *unregulated* diabetes patients, due to high blood glucose levels, diabetes patients *lose* weight. This weight loss may find its cause in the fact that when blood glucose levels rise, the glucose is excreted through urine and 'energy' is lost. Based on WO2006/009437, a *well-regulated* diabetes patient eating the same amount of calories would *not* be expected to lose weight. WO'437 is thus silent on any down-regulation of overweight through intervention.

EP1424074 discloses a composition with palatinose and/or trehalulose for controlling blood sugar level. The effect of palatinose and/or trehalulose on blood glucose levels is tested in normal mice, and, lower glucose peaks are observed. It does not teach controlling overweight in diabetes patients. In fact, it shows that its composition causes decrease in normal growth of non-diabetic 5-7 weeks old mice. A skilled person is not searching for ways to prevent *normal* growth. Figure 7 deals with weight change in time in adult diabetes mice, but no weight decrease is shown. In fact, one of its products even shows a weight increase.

WO2010/104374 discloses the use of a composition for the treatment of diabetes. Nowhere in the document is there a pointer to body weight control of diabetes patients. No effects on weight control are shown, the focus is on postprandial endocrine (hormone) response. It claims the treatment of *un*desired weight loss, i.e. prevention of weight loss which teaches away from the present invention.

There is, therefore, an urgent need for new approaches to address obesity of patients with diabetes and their associated complications.

In the field, a ready-to-drink, nutritionally complete (1 kcal/ml) supplement, specifically designed for diabetes, is commercialized as Diasip® for superior glucose control. However, a link with weight control has not been made. Both obesity and type 2 diabetes are associated with insulin resistance. However, most obese, insulin-resistant individuals do not develop hyperglycaemia. Under normal conditions, the pancreatic islet cells increase insulin release sufficiently to overcome the reduced efficiency of insulin action, thereby maintaining normal glucose tolerance. Hence, aiming to improving postprandial glucose response is not considered the same as treating weight issues.

### SUMMARY OF THE INVENTION

The inventors surprisingly found a nutritional composition which is capable to significantly decrease the body weight of diabetes patients. Obesity increases the likelihood of various diseases, particularly heart disease and type 2 diabetes. Pre-obese or obese diabetes patients strongly benefit from the decrease in body weight realized by administering the nutritional composition of the invention. Treatment or prevention of obesity in type 2 diabetes patients is therefore a crucial medical intervention. Details are provided in example 1.

Surprisingly the inventors also discovered that use of the composition according to the invention decreases the total cholesterol levels and triglyceride levels in blood. Details are provided in example 2. Since high and uncontrolled cholesterol and triglyceride levels are are believed to be linked with cardiovascular disorders in the art, and the targeted type 2 diabetes patients often suffer from, or are at high risk of developing cardiovascular disorders, in one aspect the composition also plays a role in treating cardiovascular disorders in these patients.

The invention thus pertains to a composition for use in the dietary management of cholesterol levels or triglyceride levels in a type 2 diabetes patient, wherein the cholesterol or triglyceride levels in blood are reduced; said product having an energy density between 0.9 and 1.5 kcal/ml and comprising isomaltulose and a protein source, wherein said protein source is present in between 12 and 25 en% based on the total product, and comprising at least 10 wt% of aspartate equivalents based on the total protein content.

The nutritional composition may be a meal or snack replacer.

### LIST OF PREFERRED EMBODIMENTS

In a preferred embodiment, the composition comprises 10 - 14 wt% of digestible carbohydrates (cho), and wherein said isomaltulose is present between 20 and 80 wt% of the total weight of the digestible carbohydrates.

In a preferred embodiment, the composition further comprises one or more indigestible carbohydrates selected from the group consisting of galacto-oliogsaccharides, fructo-oligosaccharides, inulin, resistant dextrin, resistant starch, cellulose, pectin, or pectin degradation products.

In a preferred embodiment, the patient to which the composition is administered has a BMI of at least 25 kg/m².

In a preferred embodiment, the protein source in the composition comprises whey and/or soy proteins, and/or their hydrolyzates, concentrates and/or isolates.

In a preferred embodiment, the composition comprises whey and soy proteins, and/or their hydrolyzates, concentrates and/or isolates, in a weight ratio ranging between 2:1 and 1:2.

In a preferred embodiment, the composition has a weight ratio of aspartate equivalents over glutamate equivalents of at least 0.45:1.

### DETAILED DESCRIPTION OF THE INVENTION

### Definitions:

For the purpose of this document, "aspartate equivalents" are L-aspartic acid and/or L-asparagine. The amino acids may be present as free amino acids or as peptides or proteins.

Unless defined otherwise, dosages given in weight percentage (wt%) aspartate equivalents correspond to the percentages of L-aspartic acid and L- asparagine per total weight of the protein in the composition. In the calculations, the residues in peptides and proteins are corrected for the lack of a water molecule in the amino acid chain.

"Obesity" is a medical condition in which excess body fat has accumulated to the extent that it may have an adverse effect on health, leading to reduced life expectancy and/or increased health problems. Worldwide, obesity has been linked to the body mass index (BMI), a measurement which compares weight and (height)². People are medically defined as overweight (pre-obese) if their BMI is between 25 kg/m² and 30 kg/m², and obese if their BMI is greater than 30 kg/m². Hence, the invention is particularly directed to patients having a BMI of at least 25 kg/m², especially patients having a BMI greater than 30 kg/m².

For the purpose of the present invention, "digestible carbohydrates" are carbohydrates that are digestible in the gastrointestinal tract by the action of digestive enzymes. Examples of digestible carbohydrates are glucose, fructose, galactose, lactose, maltose, sucrose, isomaltulose, trehalose, starches, maltodextrins, etc.

Isomaltulose also known by the trade name 'Palatinose' is a disaccharide that is commercially manufactured enzymatically from sucrose via bacterial fermentation. It is a natural constituent of honey and sugar cane and has a very natural sweet taste. Isomaltulose is fully absorbed in the small intestine as glucose and fructose. Like sucrose, it is fully digested and provides the same caloric value of approximately 4 kcal/g.

"Indigestible carbohydrates or dietary fibers" are carbohydrates that are not or essentially not digested by the gastro intestinal enzymes, but are fermented or partially fermented in the intestinal tract by the action of bacteria.

"Protein source" can be any amino acid source capable of delivering sufficient aspartate (asp) and asparagine (asn). The protein source is selected from the group consisting of proteins, peptides and/or free amino acids.

In the context of this application, the % of total energy is abbreviated as en%; en% is thus short for energy percentage and represents the relative amount that a constituent contributes to the total caloric value of the composition. The Atwater constants 9 and 4 kcal/ g dry weight are used for calculating the energy content of lipids and protein or digestible carbohydrates respectively. For nutritional fibre the energy density of 2 kcal per g dry weight is applied.

The term"meal replacer" as used herein refers to a product which is intended to replace one or more conventional meals a day as part of a weight loss or weight plan; they are of a controlled calorie content and are generally eaten as a single product; on the one hand they provide a restricted caloric intake, but on the other hand they provide a healthy balance of nutritional ingredients and are convenient to incorporate into an individual's daily diet. The term also includes products which are eaten as part of a meal replacement weight loss or weight control plan, for example snack products which are not intended to replace a whole meal by themselves but which may be used with other such products to replace a meal or which are otherwise intended to be used in the plan; these products typically have a calorie content in the range of from 50-250 kilocalories per serving.

### Embodiments

In one aspect, the invention pertains to a nutritional composition for use in dietary management of cholesterol levels or triglyceride levels in diabetes patients, wherein the cholesterol or triglyceride levels in blood are reduced, said composition comprising isomaltulose and a protein source, and said composition having an energy density between 0.7 and 1.5 kcal/ml, and wherein the protein source comprises at least 10 wt% of aspartate equivalents based on the total protein content, and wherein said protein source is present in between 12 and 25 en% based on the total composition,

Preferably the composition comprises between 30 and 60 en% digestible carbohydrates, more preferably between 35 and 55 en% and even more preferably between 40 and 50 en%. The composition according to the invention preferably further comprises between 25 and 45 en% fat, more preferably between 30 and 40 en%. The energy percentage is based on the total caloric content of the composition.

A preferred embodiment according to the present invention comprises digestible carbohydrates wherein at least part of the digestible carbohydrates is isomaltulose. The composition preferably comprises 35 - 55 wt% digestible carbohydrates based on the total dry weight of the composition. Preferably the carbohydrate fraction comprises at least 20wt% isomaltulose, even more preferably between 20 and 80 wt% isomaltulose, even more preferably at least 30 wt% isomaltulose, and most preferably between 30 and 60 wt%, in particular between 30 and 50 wt% of isomaltulose, based on the total weight of the digestible carbohydrates. If desired, the preferred isomaltulose content with regard to the total composition can be calculated from the numbers in this paragraph. In one embodiment, the composition contains up to 40 wt% lactose, more preferably between 15 and 35 wt% lactose, based on the total weight of digestible carbohydrates.

The protein source is preferably present between 14 and 23 en% and even more preferably between 17 and 21 en%, based on total caloric content of the composition. Alternatively, the protein content is between 15 - 30 wt%, preferably between 16- 25 wt%, even more preferably between 17 and 22 wt% based on the total dry weight of the composition. In a preferred embodiment, the amount of aspartate equivalents in the protein fraction is at least 10.5 wt%, more preferably at least 11 wt%, most preferably at least 11.5 wt%.

Although the invention allows for the use of free aspartate equivalents to reach the preferred minimum aspartate level, it is preferred that the protein source comprises proteins and or their hydrolyzates. The protein source is preferably whey based protein or soy based protein since these sources are especially high in Asp and Asn. Therefore, a preferred embodiment according to the present invention comprises a protein source comprising whey and/or soy proteins, including their hydrolyzates, concentrates and/or isolates. In a preferred embodiment, the composition contains whey and soy proteins (including their hydrolyzates, concentrates and/or isolates) in a weight ratio of 2:1 to 1:2. In a preferred embodiment, the weight ratio of aspartate equivalents over glutamate equivalents in the composition is at least 0.45:1. It is preferred that whey and/or soy proteins, hydrolyzates, isolates and/or concentrates make up for at least 80 wt%, more preferably at least 90 % of the total protein content.

For practical reasons and because of its beneficial amino acid composition, whey from cow's milk is particularly suitable as a protein ingredient, for example sweet whey that results after cheese manufacture or acid whey. The latter is a very suitable source due to the absence of glycomacropeptide. Very suitable whey proteins are α-lactalbumin-enriched whey proteins having a content of aspartate equivalents of at least 12 wt% and an asp:glu ratio of at least 0.58.

In one embodiment, the protein fraction comprises less than 10 wt%, preferably less than 5 wt% of casein or caseinates. In one embodiment, it is preferred that the weight ratio of whey to casein is at least 9:1.

In a preferred embodiment according to the present invention, the composition comprises one or more indigestible carbohydrates selected from the group consisting of galactooligosaccharides, fructooligosaccharides, inulin, resistant dextrin, resistant starch, cellulose, pectin, or degradation products thereof, e.g. hydrolysates. The amount of fibers or indigestible carbohydrates is preferably between 1 and 3 g per 100 ml.

Tables A and B list preferred embodiments.

**Table A. Preferred embodiments (liquid, per 200 ml)**

| **Ingredient** | **Unit** | **Preferred** | **Most preferred** |
|---|---|---|---|
| **energy** | Kcal | 150 - 250 | 200 |
| **protein** | g / en% | 8 - 12g | 9.8 / 16 |
| whey protein | g | 4 - 6 | 4.8 |
| soy protein | g | 4 - 6 | 4.8 |
| **digestible carbohydrates** | g / en% | 20 - 28g | 23.4 / 47 |
| lactose | g | 6 - 8 | 7.2 |
| isomaltulose | g | 7.5 - 10.5 | 8.8 |
| glucose | g | 0.4 - 0.8 | 0.6 |
| **fat** | g / en% | 6 - 9g | 7.6 / 34 |
| **fibre (indigestible cho)** | g | 3 - 5 | 4 |

**Table B. Preferred embodiments (powder, per 100 g dry powder*^{,}**)**

| **Ingredient** | **Unit** | **Preferred** | **Most preferred** |
|---|---|---|---|
| **energy** | Kcal | 350 - 500 | 417 |
| **protein** | g / en% | 17 - 23 g | 19.8 / 19 |
| aspartate equivalents | g | > 1.7 | 2.2 |
| **digestible carbohydrates** | g / en% | 46 - 52 g | 48.8 / 47 |
| isomaltulose | g | 20 - 40 | 27 |
| **fat** | g / en% | 13 - 19 | 15.9 /34 |
| **fibre (indigestible cho)** | g | 6 - 10 | 8.3 |

| | | | |
|---|---|---|---|
| *contains less than 4.5 g water **19.2 g powder is used to prepare 100 ml composition | | | |

The composition according to the invention is designed to either supplement a diet or to provide complete nutritional support. Hence, the composition according to the invention may further comprise fat and/or a source of vitamins, minerals and trace elements. Preferably, the composition according the invention is a nutritionally complete composition.

In a preferred embodiment the composition according to the present invention is a complete nutritional product comprising protein, fat, digestible carbohydrates and indigestible carbohydrates, wherein the protein comprises at least 10 wt% aspartate equivalents, preferably comprising aspartate and/or asparagines, and the composition contains between 12 and 25 en% protein.

With regard to the type of fat, a wide choice is possible, as long as the fat is of food quality. The composition preferably contains between 25 and 45 en% fat, more preferably between 30 and 40 en%. The fat may either be an animal fat or a vegetable fat or both. Although animal fats such as lard or butter have essentially equal caloric and nutritional values and can be used interchangeably, vegetable oils are highly preferred in the practice of the present invention due to their readily availability, ease of formulation, absence of cholesterol and lower concentrations of saturated fatty acids. In one embodiment, the present composition thus comprises rapeseed oil, corn oil and/or sunflower oil. It is preferred that a large portion of the fat fraction is provided by monounsaturated fatty acids, preferably between 10 and 30 en%, most preferably between 15 and 25 en%, of the total caloric content of the composition.

The nutritional composition is preferably administered as a discrete serving or dosage unit. Dosage units are defined as physically identifiable separate units, usually in packaged form. A tube-feed is not comprised in the term "dosage unit" in the context of the invention, given its continuous administration regime. Preferably the nutritional composition is in the form of a liquid or a powder, but can have other forms as well such as a bar.

In one embodiment of the present invention, the composition according to the invention is packaged. The packaging may have any suitable form, for example a block-shaped carton, e.g. to be emptied with a straw ; a carton or plastic beaker with removable cover; a small-sized bottle or cup for example for the 80 ml to 400 ml range, preferably 100 - 300 ml range.

The invention pertains to a composition according to the above defined features for use in the dietary management of cholesterol levels and/or triglyceride levels in type-2 diabetes patients, wherein the cholesterol and triglyceride levels in blood are reduced. In particular the composition is suitable for treating cardiovascular diseases in type-2 diabetes patients. The invention also pertains to a composition for use in the dietary management of cholesterol levels and/or triglyceride levels (i.e. managing, controling or regulating cholesterol blood levels and/or triglyceride blood levels) in a type-2 diabetes patient, wherein the cholesterol and triglyceride levels in blood are reduced, particularly for treating cardiovascular diseases in a type-2 diabetes patient, comprising orally administering a nutritional composition having one or more of the above-defined features to a patient suffering from type 2 diabetes [in need thereof]. With 'prevention' it is understood that the use involves reducing the risk (of occurence) in the targeted population.

The patient is preferably pre-obese or obese, in the medical field often characterized in terms of BMI, as discussed above.

### EXAMPLES

### Example 1 - clinical trial with diabetes patients

The effect was studied of the diabetes-specific formula comprising isomaltulose and a specific protein source comprising at least 10wt% aspartate on body weight after 4 weeks consumption by type 2 diabetes patients. Furthermore, glycemic and lipid parameters, weight and tolerance were also monitored. The formula was consumed as a breakfast and afternoon or evening snack replacer in type 2 diabetes patients.

The randomized, controlled, double-blind, parallel-group study involved forty-four type 2 diabetes patients on oral anti-diabetes medication, which were given 2x200mL/day of either a diabetes-specific formula comprising isomaltulose and a specific protein source comprising at least 10wt% aspartate, or an isocaloric standard, fiber-containing formula, over 4 weeks.

### Abbreviation

HbAlc - Glycosylated haemoglobin; HDL -High Density Lipoprotein; HOMAIR - Homeostasis model assessment for insulin resistance; iAUC - incremental Area Under the Curve; ITT -Intention-To-Treat; LDL - Low Density Lipoprotein.

### Results

Changes in body weight between groups were significantly different, with an increase in the standard group and a decrease in the treatment group. Both products were equally well tolerated.

### Materials and Methods

The study was a randomized, controlled, double-blind, parallel-group study, performed at one study center in the Netherlands (Julius Center, Utrecht, The Netherlands).

### Subjects

In total, 44 ambulatory type 2 diabetes patients (25 male, 19 female) were included in this study.

Inclusion criteria were diagnosis of type 2 diabetes according to the WHO criteria for more than 6 months, male with an age > 18 years or postmenopausal female, HbAlc between 6.5% and 8.0%, body mass index (BMI) between 18 and 35 kg/m², on a stable and controlled anti-diabetic regime for at least two months with metformin and/or sulphonylureas and regimes expected to remain stable throughout the duration of the study. If lipid-lowering drugs were used, their use was to be stable and controlled for at least two months and expected to remain stable throughout the duration of the study.

Exclusion criteria were a weight loss diet, any gastrointestinal disease that interferes with bowel function and nutritional intake significant heart (NYHA class IV), hepatic (transaminase greater than 3 times upper limit of normal), or renal disease (requiring dialysis or creatinine > 160 µmol/L), infection requiring antibiotics within 3 weeks prior to study entry, concomitant therapy with insulin or anti-diabetic medication other than metformin and sulphonylureas, therapy with systemic glucocorticoids at or within 2 weeks prior to study entry, alcohol abuse, fever and participation in other intervention studies within 4 weeks of screening. Subjects with galactosemia, lactose intolerance or subjects requiring a fiber free diet were also excluded.

The Independent Review Board Nijmegen (The Netherlands) approved the study protocol. All subjects were informed about the nature of the study and gave written informed consent prior to study screening. The study was performed in accordance with the principles relating to the Declaration of Helsinki and GCP.

### Treatments

Subjects were randomly allocated to receive either the diabetes-specific formula or an isocaloric standard formula according to a computer generated randomization list using two different randomization codes per treatment.

### Formulate

The subjects in the diabetes-specific formula group consumed a treatment formula, a flavored, 1 kcal/mL, diabetes-specific nutritional supplement (47 en% carbohydrates, 19 en% protein, 34 en% fat (20 en% monounsaturated fatty acids) and 2 g fibers/100 mL wherein the protein delivers 12 wt% aspartate + asparganine based on the total protein content).

The control group received a flavored isocaloric, fiber-containing standard formula containing 50 en% carbohydrates, 16 en% protein, 34 en% fat (20 en% monounsaturated fatty acids) and 1.5 g fibers/100 mL, and wherein the protein delivers 8wt% apartate + asparagine.

In the study, the formulas are referred to as the 'treatment' and the 'standard', respectively. Both formulas contained the same amount and quality of fat. Both formulas contained vitamins, minerals, and trace elements in accordance with the regulations for Food for Specific Medical Purposes (1999/21/EC). Subjects consumed 2 portions of 200 mL per day of either of the products for four weeks; one as a replacement of breakfast and one as an in-between snack in the afternoon or evening.

Table 1 shows the characteristics of the formula compositions.

**Table 1. Macronutrient composition of the formulas (per 100 ml). Differences**

| **Ingredient** | **Unit** | **Treatment formula** | **Control formula** |
|---|---|---|---|
| **Energy** | kcal | 100 | 100 |
| **Protein** | g / en% | 4.8 / 19 | 4.0 / 16 |
| whey protein | | 2.4 | |
| soy protein | | 2.4 | |
| casein | | - | 4.0 |
| asp equivalents | wt% | 13 | 8 |
| **Digestible Carbohydrate** | g / en% | 11.5 / 47 | 12.55 / 50 |
| lactose | | 3 | |
| isomaltulose | | 4.4 | |
| starch* | | 0.4 | 12 |
| resistant starch | | - | 0.2 |
| oligo/polysaccharides | | 3 | |
| others | | 1.1 | 0.35 |
| **Fat** | g / en% | 3.78 / 34 | 3.78 / 34 |
| **Fiber** | g | 2.0 | 1.5 |

| | | | |
|---|---|---|---|
| * Starch is present to compensate for the lack of isomaltulose in the control, thus rendering the treatment and control formulae isocaloric. The small amounts of starch present in the treatment formula are merely there because some minerals and vitamins are added in a starch matrix | | | |

### Study design

After screening, the subjects visited the research center at the beginning (visit 1, day 1)) and at the end (visit 2, day 29) of the 4-week period. Subjects had to refrain from alcohol consumption and intense physical activities for respectively 24 and 48 h before both visits and had to eat a standard meal (450 g, 125 kcal/100 g, 57 en% carbohydrates, 13 en% protein, 30 en% fat) the evening before. They were instructed to eat until they felt comfortably full. Subjects came to the study center in the morning after an overnight fast (10 h) and body temperature and weight were measured. Venous blood was collected via a canula which was placed at least 30 minutes before the first blood sample was taken. Subjects took their anti-diabetes medication with 150 mL water 20 minutes before intake of the study product. Subjects consumed either the diabetes-specific formula or the standard formula (200 mL) according to randomization and had to finish it within 5 minutes. To determine the postprandial glucose and insulin responses, venous blood samples were drawn 5 minutes prior to product intake (basal sample) and 15, 30, 45, 60, 75, 90, 120, 150, 180, 210 and 240 minutes after intake of product. Besides postprandial measurements, also fasting blood samples were collected at visit 1 and 2 for the measurement of total cholesterol, high density lipoprotein cholesterol (HDL), low density lipoprotein cholesterol (LDL) and triglycerides, and for alanine amino transferase, aspartate amino transferase, gamma glutamyl transferase, alkaline phosphatase and creatinine and serum fructosamine levels. During the assessment period subjects had to sit quietly.

During the 4 week study period, subjects filled out a diary daily on actual formula consumption. On the 4 days immediately preceding visit 1, on days 1-4, on days 15-18 and on days 25-28, a questionnaire on gastrointestinal tolerance was completed by the subjects. In this questionnaire, subjects reported the intensity of dry mouth, thirst, belching, heartburn, bloating, stomach pain, stomach cramps, abdominal pain, flatulence, nausea on a 4-point scale ranging from 'not at all' to 'very much'. Furthermore, subjects recorded defecation frequency and consistency on a 5-point scale. Adverse events were reported voluntarily to the investigator and recorded in the study data base.

### Laboratory methods

All blood samples were centrifuged immediately (1000 g, 10 min, 4°C), except for serum which was allowed to clot at room temperature (30 min). After centrifugation, plasma and serum aliquots were stored at -20°C until analysis. For the analysis of HbAlc, EDTA whole blood was collected and stored at 4°C until analysis at the same day.

Plasma glucose and triglycerides were analyzed enzymatically on a DxC (Beckman Coulter, Brea, (California), United States). Serum insulin was measured using a chemiluminescence immunoassay (E170, Roche Diagnostics, Basel, Switzerland) and serum fructosamine was measured using a colorimetric assay (Pentra 400, Horiba ABX inc., Irvine (California), United States). Plasma total cholesterol and HDL cholesterol were determined on a DxC (Beckman Coulter, Brea, (California), United States) using enzymatic colorimetric kits. LDL cholesterol was estimated using the Friedewald equation. Biochemical parameters and HbAlc were measured using standardized HPLC methods (HA-8160, Menarini, Valkenswaard, The Netherlands).

### Outcome measures

The primary outcome measure of this trial was the 4 hour postprandial plasma glucose response, as assessed by the incremental area under the curve (iAUC). As secondary outcome measures, body weight, and several other parameters were defined.

### Results

Forty-four subjects were enrolled, twenty-two per treatment group, and all these subjects were included in the ITT analyses. Forty-two subjects completed the study. In the standard formula group, one subject dropped out since he indicated that most likely, he was going to forget to take the study product twice a day. In the diabetes-specific formula group, one subject dropped out due to difficulties in placing a canula.

### Compliance

Subjects in the treatment formula and standard formula (control) group consumed on average 95.7 ± 2.33 and 92.2 ± 4.66% (p=0.79) of total daily intake (400 mL), respectively.

### Efficacy

Interestingly, the change in body weight was significantly different between groups (-0.3 ± 0.2 kg in treatment group and +0.6 ± 0.2 kg in placebo group; p= 0.006). The body weight was not significantly different at either of the visits. The effect found on weight loss were unexpected. This is surprising since both the treatment and standard formula were isocaloric and were consumed at the same time and in the same quantities.

### Example 2 - clinical trial with diabetes patients II

Based on the surprising results on body weight in the study of example 1, a second clinical study was done wherein 112 diabetes type 2 patients received the same product according to the invention, (treatment formula of table 1 of example 1) as a replacement of one meal per day for a period of 4 weeks. Weight, HbAlc, fasting blood glucose levels, blood cholesterol and triglyceride levels were measured at the start of the study and at 4 weeks after the start of the study according to the methods described in example 1. Body weight was also measured 4 weeks before the start of the study.

### Weight loss:

In the 4 weeks period before the start of the study, no significant weight loss was found (- 0.1 kg). After 4 weeks of using the product, 90% of the patients loss weight and the average weight loss was 2.1 kg (p<0.01).

### Fasting blood glucose

73% of the patients had lower fasting blood glucose levels and the average blood glucose levels decreased from 135.9 to 123.9 (-12.0) mg/dL (p<0.001).

### Cholesterol and triglyceride decrease in the blood

In the 4 weeks treatment period, the average cholesterol levels decreased significantly from 194.4 to 183.7 (-10.7) mg/dL (p<0.001) and the average blood triglyceride level decreased from 179.2 to 158.5 (-20.7) mg/dL.

### Efficacy

The results show that the treatment of type 2 diabetes patients with the composition as disclosed herein is effective in inducing weight loss and reducing cholesterol and triglyceride levels in the blood. The conclusion is that the product is effective for the nutritional management of body weight, cholesterol and triglyceride levels in diabetes patients. Diabetes patients often suffer from cardio vascular diseases. It is well established that high blood sugar levels and high cholesterol and triglyceride levels in the blood are risk factors for cardiovascular diseases. The product as disclosed herein is therefore suitable for use in the treatment of cardiovascular diseases in diabetes patients, in particular type 2 diabetes patients.

## Claims

1. A composition having an energy density between 0.9 and 1.5 kcal/ml and comprising isomaltulose and a protein source, wherein said protein source is present in between 12 and 25 en% based on the total composition, and comprising at least 10 wt% of aspartate equivalents based on the total protein content, for use in the dietary management of cholesterol levels in a type 2 diabetes patient, wherein the cholesterol levels in blood are reduced, or for use in the dietary management of triglyceride levels in a type 2 diabetes patient, wherein the triglyceride levels in blood are reduced.

2. The composition for use according to claim 1, comprising 10 - 14 wt% of digestible carbohydrates, and wherein said isomaltulose is present between 20 and 80 wt% of the total weight of the digestible carbohydrates.

3. The composition for use according to any one of the preceding claims, wherein the composition further comprises one or more indigestible carbohydrates selected from the group consisting of galacto-oliogsaccharides, fructo-oligosaccharides, inulin, resistant dextrin, resistant starch, cellulose, pectin, or pectin degradation products.

4. The composition for use according to any one of the preceding claims, wherein said patient has a BMI of at least 25 kg/m².

5. The composition for use according to any one of the preceding claims, wherein said protein source comprising whey and/or soy proteins, and/or their hydrolyzates, concentrates and/or isolates.

6. The composition for use according to claim 5, comprising whey and soy proteins, and/or their hydrolyzates, concentrates and/or isolates, in a weight ratio ranging between 2:1 and 1:2.

7. The composition for use according to any one of the preceding claims, said composition having a weight ratio of aspartate equivalents over glutamate equivalents of at least 0.45:1.

8. The composition for use according to any one of the preceding claims, said composition being a meal replacer or snack replacer.

9. The composition for use according to any of the preceding claims, wherein said type 2 diabetes patient suffers from obesity.

10. The composition for use according to any of the preceding claims, wherein said type 2 diabetes patient suffers from cardiovascular disorders.

## Patentansprüche

1. Zusammensetzung, die eine Energiedichte zwischen 0,9 und 1,5 kcal/ml aufweist und Isomaltulose und eine Proteinquelle umfasst, wobei die Proteinquelle zu zwischen 12 und 25 en-%, bezogen auf die Gesamtzusammensetzung, vorhanden ist, und wenigstens 10 Gew.-% Aspartatäquivalente, bezogen auf den Gesamtproteingehalt, umfasst, zur Verwendung in der diätetischen Behandlung der Cholesterinwerte bei einem Patienten mit Typ 2-Diabetes, wobei die Cholesterinwerte im Blut gesenkt werden, oder zur Verwendung in der diätetischen Behandlung der Triglyceridwerte bei einem Patienten mit Typ 2-Diabetes, wobei die Triglyceridwerte im Blut gesenkt werden.

2. Zusammensetzung zur Verwendung nach Anspruch 1, die 10-14 Gew.-% verdauliche Kohlenhydrate umfasst, und wobei die Isomaltulose zu zwischen 20 und 80 Gew.-% des Gesamtgewichts der verdaulichen Kohlenhydrate vorhanden ist.

3. Zusammensetzung zur Verwendung nach einem der vorangehenden Ansprüche, wobei die Zusammensetzung ferner ein oder mehrere unverdauliche Kohlenhydrate umfasst, die ausgewählt sind aus der Gruppe bestehend aus Galactooligosacchariden, Fructooligosacchariden, Inulin, resistentem Dextrin, resistenter Stärke, Cellulose, Pektin oder Pektinabbauprodukten.

4. Zusammensetzung zur Verwendung nach einem der vorangehenden Ansprüche, wobei der Patient einen BMI von wenigstens 25 kg/m² aufweist.

5. Zusammensetzung zur Verwendung nach einem der vorangehenden Ansprüche, wobei die Proteinquelle Molken- und/oder Sojaproteine und/oder ihre Hydrolysate, Konzentrate und/oder Isolate umfasst.

6. Zusammensetzung zur Verwendung nach Anspruch 5, die Molken- und Sojaproteine und/oder ihre Hydrolysate, Konzentrate und/oder Isolate in einem Gewichtsverhältnis im Bereich zwischen 2:1 und 1:2 umfasst.

7. Zusammensetzung zur Verwendung nach einem der vorangehenden Ansprüche, wobei die Zusammensetzung ein Gewichtsverhältnis von Aspartatäquivalenten zu Glutamatäquivalenten von wenigstens 0,45:1 aufweist.

8. Zusammensetzung zur Verwendung nach einem der vorangehenden Ansprüche, wobei die Zusammensetzung ein Mahlzeitersatz oder Zwischenmahlzeitersatz ist.

9. Zusammensetzung zur Verwendung nach einem der vorangehenden Ansprüche, wobei der Patient mit Typ 2-Diabetes an Fettleibigkeit (Obesitas) leidet.

10. Zusammensetzung zur Verwendung nach einem der vorangehenden Ansprüche, wobei der Patient mit Typ 2-Diabetes an Herz-Kreislauf-Erkrankungen leidet.

## Revendications

1. Composition ayant une densité d'énergie comprise entre 0,9 et 1,5 kcal/ml et comprenant de l'isomaltulose et une source de protéines, dans laquelle ladite source de protéines est présente entre 12 et 25 % en valeur énergétique sur la base de la composition totale, et comprenant au moins 10 % en poids d'équivalents d'aspartate sur la base de la teneur totale en protéines, pour une utilisation dans la gestion diététique de taux de cholestérol chez un patient atteint du diabète de type 2, dans laquelle les taux de cholestérol dans le sang sont réduits, ou pour une utilisation dans la gestion diététique de taux de triglycérides chez un patient atteint du diabète de type 2, dans laquelle les taux de triglycérides dans le sang sont réduits.

2. Composition pour utilisation selon la revendication 1, comprenant 10 à 14 % en poids de glucides digestibles, et dans laquelle ladite isomaltulose est présente entre 20 et 80 % en poids du poids total des glucides digestibles.

3. Composition pour utilisation selon l'une quelconque des revendications précédentes, dans laquelle la composition comprend en outre un ou plusieurs glucides non-digestibles choisis parmi le groupe constitué de galacto-oliogsaccharides, fructo-oligosaccharides, inuline, dextrine résistante, amidon résistant, cellulose, pectine, ou produits de dégradation de pectine.

4. Composition pour utilisation selon l'une quelconque des revendications précédentes, dans laquelle ledit patient a un BMI d'au moins 25 kg/m².

5. Composition pour utilisation selon l'une quelconque des revendications précédentes, dans laquelle ladite source de protéines comprend des protéines de lactosérum et/ou de soja, et/ou leurs hydrolysats, concentrés et/ou isolats.

6. Composition pour utilisation selon la revendication 5, comprenant des protéines de lactosérum et de soja, et/ou leurs hydrolysats, concentrés et/ou isolats, selon un rapport en poids dans la plage comprise entre 2:1 et 1:2.

7. Composition pour utilisation selon l'une quelconque des revendications précédentes, ladite composition ayant un rapport en poids entre des équivalents d'aspartate et des équivalents de glutamate d'au moins 0,45:1.

8. Composition pour utilisation selon l'une quelconque des revendications précédentes, ladite composition étant un substitut alimentaire ou un coupe-faim.

9. Composition pour utilisation selon l'une quelconque des revendications précédentes, dans laquelle ledit patient atteint du diabète de type 2 souffre d'obésité.

10. Composition pour utilisation selon l'une quelconque des revendications précédentes, dans laquelle ledit patient atteint du diabète de type 2 souffre de troubles cardiovasculaires.
